# EUROPÄISCHE PATENTSCHRIFT

(11) **EP 2 162 418 B1**
(45) Veröffentlichungstag und Bekanntmachung des Hinweises auf die Patenterteilung: **11.11.2015**
(21) Anmeldenummer: 08760728.9
(22) Anmeldetag: 09.06.2008
(51) Int. Cl.: C07C 45/82, C07C 47/21, B01D 3/14, C07C 45/83

(54) **Kontinuierliches Verfahren zur Herstellung von Neral in reiner Form**
Continuous process for preparing neral in pure form
Procédé continu de fabrication de néral sous forme pure

(30) Priorität: 26.06.2007 EP 07111080
(43) Veröffentlichungstag der Anmeldung: 17.03.2010
(73) Patentinhaber: BASF SE, 67056 Ludwigshafen (DE)
(72) Erfinder: HEYDRICH, Gunnar, 67117 Limburgerhof (DE); KASHANI-SHIRAZI, Nawid, 68549 Ilvesheim (DE); JÄKEL, Christoph, 67117 Limburgerhof (DE); SCHMIDT-LEITHOFF, Joachim, 68165 Mannheim (DE)
(86) Internationale Anmeldenummer: PCT/EP2008/057164
(87) Internationale Veröffentlichungsnummer: WO 2009/000634

(56) Entgegenhaltungen:
- DE-A1- 10 223 971
- DE-A1- 10 223 974
- DE-A1- 10 330 934

## Beschreibung

Die vorliegende Erfindung betrifft ein kontinuierliches Verfahren zur Herstellung von Neral (cis-Citral) in reiner Form durch destillative Abtrennung von Neral aus Stoffgemischen die im wesentlichen Neral und Geranial (trans-Citral) enthalten. Dabei wird die destillative Abtrennung in einer Trennwandkolonne oder in einer Zusammenschaltung von zwei Destillationskolonnen in Form einer thermischen Kopplung vorgenommen.

Citral (3,7-Dimethyl-octa-2,6-dienal) stellt eine wichtige Aromachemikalie sowie ein Ausgangs- bzw. Zwischenprodukt zur Herstellung einer breiten Vielfalt von Wert- bzw. Wirkstoffen dar. Dabei ist insbesondere bedeutsam, dass es sich bei Citral um einen α,β-ungesättigten Aldehyd handelt, der zu einen als Synthesebaustein attraktiv ist, zum anderen auf Grund seiner hohen Reaktivität jedoch auch zu Nebenreaktionen, beispielsweise zu Isomerisierungen neigt. Citral kann sowohl aus natürlichen Quellen isoliert als auch synthetisch hergestellt werden und fällt üblicherweise in Form von Gemischen der E/Z-Isomeren Neral und Geranial an. Für spezielle Anwendungen im Bereich der Aroma- oder Synthesechemie kann es wünschenswert sein, die beiden genannten Doppelbindungsisomere in reiner oder angereicherter Form einsetzen zu können.

Problematisch ist dabei, dass die in möglichst reiner Form einzusetzenden Verbindungen Geranial und Neral sich nur durch die Konfiguration der zur jeweiligen Aldehydgruppe konjugierten ethylenischen Doppelbindung unterscheiden und leicht durch thermische Isomerisierung derselben ineinander umgewandelt werden können. Dadurch stellt die effiziente Trennung von Geranial- und Neral haltigen Gemischen im technischen Maßstab ein nach wie vor nicht zufrieden stellend gelöstes technisches Problem dar.

Die EP 1 514 955 betrifft ein Verfahren zur destillativen Aufarbeitung des Elektrolyseaustrages der elektrochemischen Oxidation von 1,1,2,2-Tetramethoxyethan mit Methanol zu Trimethylorthoformiat in einem flüssigen Elektrolyten, wobei eine Trennwandkolonne mit 30 bis 150 theoretische Trennstufen eingesetzt wird.

Die DE 103 30 934 offenbart ein Verfahren zur kontinuierlichen Isolierung von Citronellal oder Citronellol aus einem mindestens eine dieser Verbindungen enthaltenden Rohgemisch durch Rektifikation. Dabei werden vorzugsweise solche Ausgangsgemische eingesetzt, die durch partielle Hydrierung von Citral bzw. Citronellal erhalten sind. Die DE 102 23 974 betrifft ein Verfahren zur kontinuierlichen Isolierung zweier stereoisomerer Isoprenoidalkohole, speziell Nerol und Geraniol, aus einem Rohgemisch durch Rektifikation, wobei das Rohgemisch seitlich in eine Zulaufkolonne eingeführt wird, man wenigstens eine mit der Zulaufkolonne gekoppelte Abzugskolonne vorsieht und aus der Abzugskolonne einen ersten und einen zweiten Isoprenoidalkohol abzieht. Dabei werden die Zulauf- und die Abzugskolonne so gekoppelt, dass zumindest im Bereich des Abzugs der Isoprenoidalkohole keine Quervermischung von Brüden und Kondensat erfolgt.

Die DE 102 23 971 offenbart ein Verfahren zur kontinuierliche Isolierung eines α,β-ungesättigten Aldehyds, speziell Citral aus einem diesen bzw. dieses enthaltenden Rohgemisch durch Rektifikation. Dabei wird das Rohgemisch seitlich in eine Zulaufsäule mit oberhalb der Zulaufstelle gelegenem Verstärkungsteil und unterhalb der Zulaufstelle gelegenem Abtriebsteil eingeführt, eine mit dem oberen Ende des Verstärkungsteils kommuizierende obere Vereinigungssäule mit Kondensator am oberen Säulen-ende und eine mit dem unteren Ende des Abtriebsteils kommunizierende Vereinigungssäule mit Aufheizer am unteren Säulenende vorgesehen und eine mit der oberen Vereinigungssäule und der unteren Vereinigungssäule kommunizierende Vereinigungssäule vorgesehen. Der α,β-ungesättigte Aldehyd wird als Seitenabzug aus der Abzugssäule abgezogen und am Kopf der oberen Vereinigungssäule niedriger siedende Verbindungen und im Sumpf der unteren Vereinigungssäule höher siedende Verbindungen abgezogen.

In keiner der genannten Schriften wird die Trennung stereoisomerer α,β-ungesättigter Aldehyde beschrieben. Diese weisen im Vergleich zu den in der DE 102 23 974 genannten stereoisomeren α,β-ungesättigten Alkoholen eine erhöhte Reaktivität, speziell eine leichtere Isomerisierbarkeit auf. Die DE 102 23971 offenbart ebenfalls keine Trennung stereoisomerer α,β-ungesättigter Aldehyde, sondern lediglich die Abtrennung eines Gemisches von cis- und trans-Citral von anderen chemischen Verbindungen.

K. Bauer et al. erläutern in einem Standardwerk der Riechstoffliteratur (K. Bauer, D. Garbe, H. Surburg:" Common Fragrance and Flavor Materials - Preparation, Properties and Uses", Fourth Completely Revised Edition, 2001, Wiley-VCH, S. 36-38), dass Citral, also Gemische von Neral und Geranial als α,β-ungesättigte Aldehyde mit zusätzlicher Doppelbindung sehr reaktiv sind und Reaktionen wie Cyclisierung und Polymerisation eingehen. Die Trennung von cis und trans Isomeren voneinander ist durch die Bildung von Hydrogensulfit Additionsverbindungen möglich.

Jeff Sacks et al. beschreibt in einer wissenschaftlichen Abhandlung zur Auftrennung und Analyse von Citral Isomeren (Jeff Sacks, Erin Greenley, Greg Leo, Paul Willey, David Gallis, John A. Mangravite: "Separation and Analysis of Citral Isomers", Journal of Chemical Education, 60 (5), 1983, S.434 - 436) wie Geranial und Neral durch eine Vakuum-Spinning Band-Destillation getrennt werden und im Fortgang der Reaktion der Anteil an Neral in den gewonnenen Fraktionen gegenüber Geranial immer geringer wird.

Ausgehend von diesem Stand der Technik bestand die Aufgabe der vorliegenden Verbindung in der Bereitstellung eines Verfahrens zur Herstellung von möglichst reinem bzw. angereichertem Neral (cis-Citral) ausgehend von wohlfeil zugänglichem Citral. Das Verfahren soll mit apparativ geringem Aufwand wirtschaftlich und in technischem Maßstab durchführbar sein und dabei insbesondere nur in geringem Ausmaß zur Bildung von Zersetzungs- bzw. Nebenprodukten führen, d.h. das gewünschte Produkt in hoher Reinheit und in möglichst hoher Ausbeute liefern.

Die Aufgabe wurde in überraschender Weise erfindungsgemäß gelöst durch die Bereitstellung eines kontinuierlichen Verfahrens zur Herstellung von Neral der Formel (I) 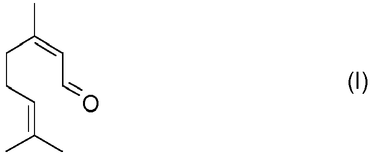 in reiner Form mit einem Neral-Gehalt von größer oder gleich 95 Gew. % bezogen auf die Gesamtmenge durch destillative Abtrennung von Neral aus Stoffgemischen enthaltend Neral und Geranial der Formel (II) 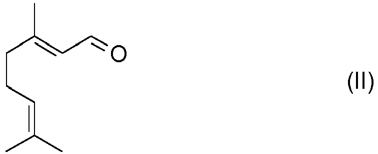 wobei man die destillative Abtrennung in einer Trennwandkolonne oder in einer Zusammenschaltung von zwei Destillationskolonnen in Form einer thermischen Kopplung mit 80 bis 200 theoretischen Trennstufen und einer oder mehrerer Seitenabzugsstellen bei einem absoluten Betriebsdruck von 5 bis 200 mbar durchführt, dadurch gekennzeichnet, dass man ein Stoffgemisch einsetzt, dass zu 30 bis 70 Gew. % aus Neral, zu 70 bis 30 Gew.-% aus Geranial und zu 0 bis 5 Gew.-% aus weiteren Komponenten besteht, wobei sich die Prozentangaben zu 100 Gew.-% addieren.

Als Einsatzstoffe zur Durchführung des erfindungsgemäßen Verfahrens eignen sich Stoffgemische die Neral und Geranial enthalten, bevorzugt solche, die zum überwiegenden Teil aus den Doppelbindungsisomeren Neral und Geranial bestehen. Bevorzugt sind darunter solche Stoffgemische, die 90 Gew.-% bis 100 Gew.-%, besonders bevorzugt 95 bis 98 Gew.-% (jeweils bezogen auf die Gesamtmenge des jeweiligen Stoffgemisches) Geranial und Neral enthalten bzw. zu den genannten Anteilen aus denselben bestehen und daneben in geringem Ausmaß, d.h. zu einem Anteil von bis zu 10 Gew.-%, bevorzugt bis zu 5 Gew.-% (jeweils bezogen auf die Gesamtmenge des jeweiligen Stoffgemisches) noch weitere Komponenten wie beispielsweise Isomere, Nebenprodukte oder Verunreinigungen enthalten können. Ein bevorzugter Einsatzstoff ist synthetisch hergestelltes Citral, insbesondere solches, dass durch thermische Spaltung von 3-Methyl-2-buten-1-al-diprenylacetal unter Abspaltung von Prenol zu cis/trans-Prenyl-(3-methyl-butadienyl)-ether, Claisen-Umlagerung desselben zu 2,4,4-trimethyl-3-formyl-1,5-hexadien und anschließende Cope-Umlagerung desselben, wie beispielsweise in der EP 0 992 477 beschrieben, erhalten wurde. Dieses enthält typischerweise etwa 45 bis etwa 55 Gew.-% Neral neben etwa 55 bis etwa 45 Gew.-% und etwa 1 bis 5 Gew.-% weiterer Verbindungen bzw. Verunreinigungen.

Im Rahmen des erfindungsgemäßen Verfahrens setzt man ein Stoffgemisch ein, dass 30 bis 70 Gew.-%, bevorzugt 40 bis 60 Gew.-% aus Neral, 70 bis 30 Gew.-%, bevorzugt 60 bis 40 Gew.-% aus Geranial und 0 bis 5 Gew.-% weitere Komponenten enthält, wobei sich die Prozentangaben zu 100 Gew.-% addieren.

Die erfindungsgemäße destillative Abtrennung wird üblicherweise so durchgeführt, dass man das eingesetzte Neral und Geranial enthaltende Stoffgemisch in jeweils eine oder mehrere Leichtsieder-, Mittelsieder- und Schwersiederfraktion bzw. -fraktionen auftrennt und Neral in reiner Form mit einem Neral-Gehalt von größer oder gleich 95 Gew. % bezogen auf die Gesamtmenge als Mittelsiederfraktion an der Seitenabzugsstelle der eingesetzten Trennwandkolonne oder der Zusammenschaltung von zwei Destillationskolonnen in Form einer thermischen Kopplung in flüssiger oder gasförmiger Form entnimmt.

Bei dem erfindungsgemäßen Verfahren handelt es sich demnach auch um ein kontinuierliches Verfahren zur Isolierung von Neral, vorzugsweise um ein kontinuierliches Verfahren zur Isolierung von Neral in reiner Form mit einem Neral-Gehalt von größer oder gleich 95 Gew. % bezogen auf die Gesamtmenge durch destillative Abtrennung von Neral aus Stoffgemischen enthaltend Neral und Geranial, wobei man die destillative Abtrennung in einer Trennwandkolonne oder in einer Zusammenschaltung von zwei Destillationskolonnen in Form einer thermischen Kopplung mit 80 bis 200 theoretischen Trennstufen und einer oder mehrerer Seitenabzugsstellen bei einem absoluten Betriebsdruck, d.h. bei einem absoluten Druck in der Trennwandkolonne oder der Zusammenschaltung von zwei Destillationskolonnen in Form einer thermischen Kopplung von 5 bis 200 mbar, bevorzugt von 5 bis 100 mbar durchführt.

Die erfindungsgemäß einzusetzende Trennwandkolonne bzw. die Zusammenschaltung von zwei Destillationskolonnen in Form einer thermischen Kopplung weist bzw. weisen 80 bis 200, bevorzugt 100 bis 180 theoretische Trennstufen und eine oder mehrere, bevorzugt 1 bis 5, besonders bevorzugt 1 bis 3 und ganz besonders bevorzugt 1 oder 2 Seitenabzugsstellen auf. Erfindungsgemäß bevorzugt setzt man eine wie vorstehend beschriebene Trennwandkolonne ein.

Das erfindungsgemäße Verfahren wird bei einem absoluten Betriebsdruck in der Trennwandkolonne bzw. in der Zusammenschaltung von zwei Destillationskolonnen in Form einer thermischen Kopplung von 5 bis 200 mbar, bevorzugt von 5 bis 100 mbar, besonders bevorzugt von 5 bis 70 mbar, ganz besonders bevorzugt von von 10 bis 50 mbar und insbesondere bevorzugt von 10 bis 40 mbar durchführt. Bevorzugt betreibt man dabei die Trennwandkolonne oder die Zusammenschaltung von zwei Destillationskolonnen in Form einer thermischen Kopplung so, dass der absolute Kopfdruck 10 bis 50 mbar, bevorzugt 10 bis 40 mbar beträgt. Ebenfalls bevorzugt betreibt man dabei die Trennwandkolonne oder die Zusammenschaltung von zwei Destillationskolonnen in Form einer thermischen Kopplung so, dass der absolute Sumpfdruck 5 bis 200 mbar, bevorzugt 10 bis 100 und besonders bevorzugt 20 bis 50 mbar beträgt.

Das Rücklaufverhältnis kann bei der Durchführung des erfindungsgemäßen Verfahrens in breiten Grenzen variiert werden und liegt üblicherweise bei etwa 5 zu 1 bis etwa 2000 zu 1, bevorzugt etwa 20 zu 1 bis 1000 zu 1. Vorteilhaft ist auch eine Dephlegmator-Fahrweise, d.h. es wird im Kopfkondensator der Kolonne nur der Rücklauf kondensiert und wieder der Kolonne zugeführt. In einem solchen energetisch günstigen Fall der Teilkondensation fällt das auszuschleusende Kopfprodukt ausschließlich im Nachkühler an, der bei niedrigerer Temperatur betrieben werden kann.

Unter dem Begriff Neral in angereicherter Form sind Neral-haltige Stoffgemische zu verstehen, die einen höheren Gehalt an Neral aufweisen als das jeweils erfindungsgemäß eingesetzte Neral oder Geranial enthaltende Stoffgemisch. Bevorzugt ist unter dem Begriff Neral in angereicherter Form solches Neral zu verstehen, dass eine Reinheit, d.h. einen Neral-Gehalt von 80 bis 95 Gew.-%, bevorzugt von 85 bis 95 Gew.-% und ganz besonders bevorzugt von 90 bis 95 Gew.-% (jeweils bezogen auf die Gesamtmenge) aufweist. Das erfindungsgemäße Verfahren ermöglicht auch die Herstellung von Neral (cis-Citral) in reiner Form. Unter dem Begriff Neral in reiner Form ist Neral mit einem Gehalt von größer oder gleich 95, 96 oder 97 Gew.-%, bevorzugt größer oder gleich 98 Gew.-% und besonders bevorzugt 98 bis 99,5 Gew.-% zu verstehen. Insbesondere bevorzugt ist unter dem Begriff Neral in reiner Form solches Neral zu verstehen, dass einen Geranial-Gehalt von bis zu 1 Gew.-% bevorzugt von 0,05 bis 0,5 Gew.-% und besonders bevorzugt von 0,1 bis 0,3 Gew.-% aufweist. Ebenfalls bevorzugt weist das erfindungsgemäß zugängliche Neral in reiner Form einen Gehalt an Isocitralen der Formeln (III), (IV) und (V) 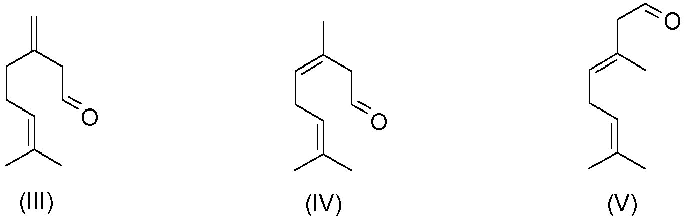 von bis zu 2 Gew.-%, bevorzugt von 0,1 bis 1 Gew.-% auf, wobei sich alle Angaben im Rahmen der vorliegenden Erfindung auf die Gesamtmenge der jeweiligen Stoffgemische beziehen.

Der Zulauf, d.h. das einzusetzende Stoffgemisch kann flüssig oder gasförmig in die Trennwandkolonne bzw. die Zusammenschaltung von zwei Destillationskolonnen in Form einer thermischen Kopplung, bevorzugt in die Trennwandkolonne geführt und dort in eine Kopf- und Sumpffraktion sowie einen oder mehrere, bevorzugt in mehrere Seitenabläufe wie vorstehend beschrieben aufgetrennt werden. In einem Seitenablauf fällt das Wertprodukt Neral in der gewünschten Reinheit an. In einer besonderen Ausführungsform ist dem Kopfkondensator der Kolonne ein Nachkondensator nachgeschaltet, der mit Kühlflüssigkeit (beispielsweise Sole) gekühlt ist und in dem noch eine Neral-arme Leichtsiederfraktion anfällt.

Für die kontinuierliche destillative Zerlegung von Mehrstoffgemischen sind nach dem Stand der Technik verschiedene Verfahrensvarianten gebräuchlich. Im einfachsten Fall wird das Zulaufgemisch in zwei Fraktionen, eine leichtsiedende Kopffraktion und eine schwersiedende Sumpffraktion, zerlegt. Bei der Auftrennung von Zulaufgemischen in mehr als zwei Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein. Die Anwendungsmöglichkeit von Destillationskolonnen mit Seitenabzügen ist jedoch dadurch stark eingeschränkt, dass die an den Seitenabzugsstellen entnommenen Produkte nie völlig rein sind. Bei Seitenentnahmen im Verstärkungsteil, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil, die meist dampfförmig erfolgen, bei denen das Seitenprodukt noch Hochsiederanteile aufweist. Die Verwendung von konventionellen Seitenabzugskolonnen ist daher auf Fälle begrenzt, in denen verunreinigte Seitenprodukte zulässig sind.

Eine Abhilfemöglichkeit bieten Trennwandkolonnen. Dieser Kolonnentyp ist beispielsweise beschrieben in US 2,471,134; US 4,230,533; EP 0 122 367; EP 0 126 288; EP 0 133 510; Chem. Eng. Technol. 10 (1987) 92 - 98; Chem.-Ing.-Tech. 61 (1989) Nr.1, 16 - 25; Gas Separation and Purification 4 (1990) 109 - 114; Process Engineering 2 (1993) 33 - 34; Trans IChemE 72 (1994) Part A 639 - 644 und Chemical Engineering 7 (1997) 72 - 76.

Bei dieser Bauart ist es möglich, Seitenprodukte ebenfalls in reiner Form zu entnehmen. Im mittleren Bereich oberhalb und unterhalb der Zulaufstelle und der Seitenentnahme ist eine Trennwand angebracht, die den Zulaufteil gegenüber dem Entnahmeteil abdichtet und in diesem Kolonnenteil eine Quervermischung von Flüssigkeits- und Brüdenströmen unterbindet. Hierdurch verringert sich bei der Auftrennung von Vielstoffgemischen die Zahl der insgesamt benötigten Destillationskolonnen. Da dieser Kolonnentyp eine apparative Vereinfachung von thermisch gekoppelten Destillationskolonnen darstellt, weist er darüber hinaus auch einen besonders niedrigen Energieverbrauch auf. Eine Beschreibung von thermisch gekoppelten Destillationskolonnen, die in verschiedener apparativer Ausgestaltung ausgeführt sein können, findet sich ebenfalls in den oben genannten Stellen in der Fachliteratur. Trennwandkolonnen und thermisch gekoppelte Kolonnen bieten gegenüber der Anordnung von konventionellen Destillationskolonnen sowohl hinsichtlich des Energiebedarfs als auch der Investitionskosten Vorteile und werden daher zunehmend industriell eingesetzt.

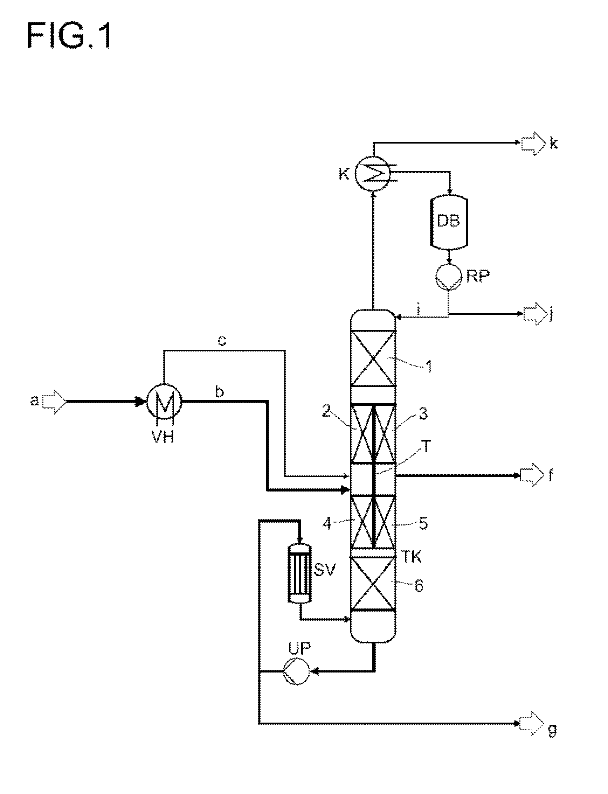

Figur 1 zeigt schematisch eine bevorzugte Ausführungsform der erfindungsgemäßen Auftrennung des einzusetzenden Neral und Geranial enthaltenden Stoffgemisches in eine Neral-arme Kopffraktion (j), eine Neral-reiche Seitenfraktion (f) und eine Neral-arme Sumpffraktion (g). Der Neral- und Geranial-haltige Zulauf zur Trennwandkolonne kann flüssig (b) gasförmig (c), bzw. gasförmig und flüssig erfolgen.

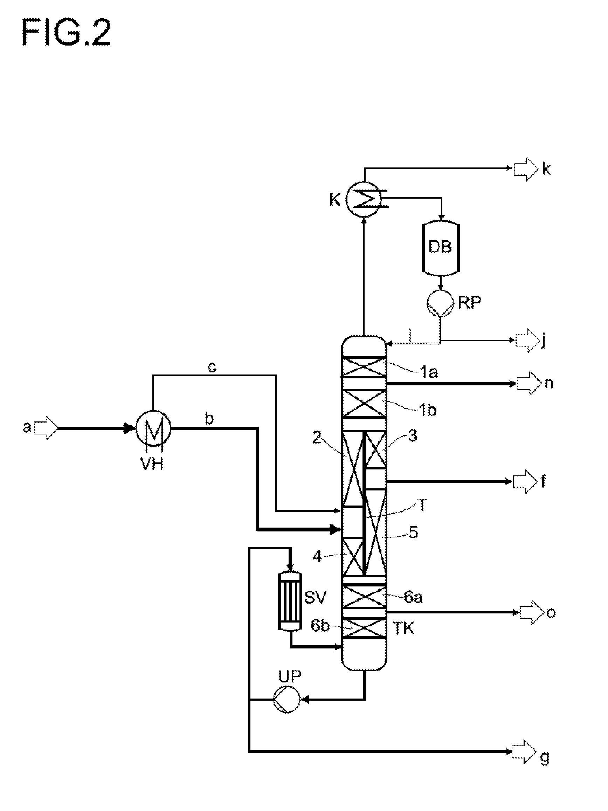

Figur 2 zeigt schematisch eine besonders bevorzugte Ausführungsform des erfindungsgemäßen Verfahrens zur Herstellung von Neral in reiner Form mit einem Neral-Gehalt von größer oder gleich 95 Gew. % bezogen auf die Gesamtmenge, bei der zusätzlich zu den unter Figur 1 genannten Merkmalen einschließlich des Seitenabzugs (f) die Seitenabzugsstellen (n) und (o) vorgesehen sind.

Das erfindungsgemäße Verfahren wird kontinuierlich durchgeführt. Demzufolge werden die als Ausgangsstoff einzusetzenden Neral und Geranial enthaltenden Stoffgemische der Trennwandkolonne oder der Zusammenschaltung von zwei Destillationskolonnen in Form einer thermischen Kopplung kontinuierlich zugeführt und die erfindungsgemäß erhaltenen Produkte (Fraktionen) bzw. Nebenprodukte kontinuierlich ausgetragen.

Der Kolonne wird üblicherweise ein weiterer Kondensator nachgeschaltet, dessen Arbeitstemperatur 10 bis 40 K, bevorzugt 20 bis 30 K unter der Arbeitstemperatur des Kopfkondensators der Trennwandkolonne liegt. Mit dessen Hilfe kann ein Großteil der noch im Kopfstrom (k) enthaltenen Leichtsieder niedergeschlagen werden.
Trennwandkolonnen können auch durch jeweils zwei thermisch gekoppelte Kolonnen ersetzt werden. Dies ist vor allem dann günstig, wenn die Kolonnen schon vorhanden sind oder die Kolonnen bei verschiedenen Drücken betrieben werden sollen. Bei thermisch gekoppelten Kolonnen kann es von Vorteil sein, den Sumpfstrom der ersten Kolonne in einem zusätzlichen Verdampfer teilweise oder vollständig zu verdampfen und danach der zweiten Kolonne zuzuführen. Diese Vorverdampfung bietet sich insbesondere dann an, wenn der Sumpfstrom der ersten Kolonne größere Mengen an Mittelsieder enthält. In diesem Fall kann die Vorverdampfung auf einem niedrigeren Temperaturniveau erfolgen und der Verdampfer der zweiten Kolonne entlastet werden. Weiterhin kann durch diese Maßnahme der Abtriebsteil der zweiten Kolonne wesentlich entlastet werden. Der vorverdampfte Strom kann dabei der zweiten Kolonne zweiphasig oder in Form von zwei separaten Strömen zugeführt werden.

Darüber hinaus kann es sowohl bei Trennwandkolonnen als auch bei thermisch gekoppelten Kolonnen von Vorteil sein, den Zulaufstrom einer Vorverdampfung zu unterziehen und anschließend zweiphasig oder in Form von zwei Strömen der Kolonne zuzuführen. Diese Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Trennwandkolonnen und thermisch gekoppelte Kolonnen können sowohl als Packungskolonnen mit Füllkörpern oder geordneten Packungen oder als Bodenkolonnen ausgeführt werden. Bei dem erfindungsgemäßen Verfahren zur Herstellung von Neral in reiner Form mit einem Neral-Gehalt von größer oder gleich 95 Gew. % bezogen auf die Gesamtmenge setzt man bevorzugt Packungskolonnen ein. Dabei sind geordnete Blech- oder Gewebepackungen mit einer spezifischen Oberfläche von etwa 100 bis 750 m²/m³, bevorzugt etwa 350 bis 500 m²/m³ besonders geeignet.

Falls, wie im Fall der vorliegenden Erfindung, besonders hohe Anforderungen an die Reinheiten der Produkte gestellt werden, ist es günstig, die Trennwand mit einer thermischen Isolierung auszustatten. Eine Beschreibung der verschiedenen Möglichkeiten der thermischen Isolierung der Trennwand findet sich in EP-A 0 640 367. Eine doppelwandige Ausführung mit einem zwischenliegenden engen Gasraum ist besonders günstig.

Für die Regelung von Trennwandkolonnen und thermisch gekoppelten Kolonne wurden verschiedene Regelungsstrategien beschrieben. Beschreibungen finden sich in US 4,230,533; DE 35 22 234 ; EP 0 780 147; Process Engineering 2 (1993) 33 - 34 und Ind. Eng. Chem. Res. 34 (1995), 2094 - 2103.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelsiederfraktion. Hierbei werden entweder einzelne für das Trennproblem kritische Komponenten, so genannte Schlüsselkomponenten, oder die Summe von mehreren Schlüsselkomponenten spezifiziert. Diese Schlüsselkomponenten sind im Rahmen der vorliegenden Erfindung Geranial als hochsiedende Nebenkomponente und Iso-Citral bzw. ein Gemisch von isomeren Iso-Citralen als tiefsiedende Nebenkomponente.

Die Einhaltung der Spezifikation für die Hochsieder in der Mittelsiederfraktion kann beispielsweise über das Aufteilungsverhältnis der Flüssigkeit am oberen Ende der Trennwand geregelt werden. Dabei wird das Aufteilungsverhältnis der Flüssigkeit am oberen Ende der Trennwand vorzugsweise so eingestellt, dass die Konzentration der Schlüsselkomponenten für die Hochsiederfraktion in der Flüssigkeit am oberen Ende der Trennwand 10 bis 80%, bevorzugt 30 bis 50%, des Wertes ausmacht, der im Seitenabzugsprodukt erzielt werden soll. Die Flüssigkeitsaufteilung wird vorzugsweise dahingehend eingestellt, dass bei höheren Gehalten an Schlüsselkomponenten der Hochsiederfraktion mehr und bei niedrigeren Gehalten an Schlüsselkomponenten der Hochsiederfraktion weniger Flüssigkeit auf den Zulaufteil geleitet wird.

Entsprechend kann die Spezifikation für die Leichtsieder in der Mittelsiederfraktion durch die Heizleistung geregelt werden. Hierbei wird beispielsweise die Heizleistung im Verdampfer so eingestellt, dass die Konzentration an Schlüsselkomponenten der Leichtsiederfraktion in der Flüssigkeit am unteren Ende der Trennwand 10 bis 80, bevorzugt 30 bis 50% des Wertes ausmacht, der im Seitenabzugsprodukt erzielt werden soll. Die Heizleistung wird vorzugsweise dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Zur Kompensation von Störungen der Zulaufmenge oder der Zulaufkonzentration erwies es sich zudem als vorteilhaft, durch einen entsprechenden Regelmechanismus (z.B. durch Regelvorschriften im Prozessleitsystem) sicherzustellen, dass die Mengenströme der Flüssigkeiten, die auf die Kolonnenteile (2), d.h. den Verstärkungsteil des Zulaufteils, und (5), d.h. den Abtriebsteil des Entnahmeteils, nicht unter 30 % ihres Normalwertes sinken können.

Zur Entnahme und Aufteilung der Flüssigkeiten am oberen Ende der Trennwand und an der bzw. den Seitenentnahmestelle(n) eignen sich sowohl innen liegende als auch außerhalb der Kolonne angeordnete Auffangräume für die Flüssigkeit, welche die Funktion einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglichen. Bei der Verwendung von gepackten Kolonnen wird die Flüssigkeit zunächst in Sammlern gefasst und von dort aus in einen innenliegenden oder außenliegenden Auffangraum geleitet.

Anstelle einer Trennwandkolonne - die bei einem Neubau hinsichtlich der Investitionskosten zu bevorzugen ist - ist es auch möglich, zwei Destillationskolonnen nach Art einer thermischen Kopplung so zu verschalten, dass sie hinsichtlich des Energiebedarfs einer Trennwandkolonne entsprechen. Sie können bei Verfügbarkeit von bestehenden Kolonnen eine sinnvolle Alternative zu Trennwandkolonnen sein. Je nach der Trennstufenzahl der vorhandenen Kolonnen können die geeigneten Formen der Zusammenschaltung ausgewählt werden.

Setzt man im Rahmen dieser Ausführungsform des erfindungsgemäßen Verfahrens zwei Destillationskolonnen in einer Zusammenschaltung in Form einer thermischen Kopplung ein, hat es sich als vorteilhaft erwiesen, beide auf diese Weise thermisch gekoppelten Destillationskolonnen jeweils mit einem eigenen Verdampfer und Kondensator auszustatten. Daneben können die beiden thermisch gekoppelten Kolonnen bei verschiedenen Drücken betrieben werden und in den Verbindungsströmen zwischen den beiden Kolonnen nur Flüssigkeiten gefördert werden. Im Rahmen einer bevorzugten Ausführungsform wird der Sumpfstrom der ersten Kolonne in einem zusätzlichen Verdampfer teilweise oder vollständig verdampft und anschließend der zweiten Kolonne zweiphasig oder in Form eines gasförmigen und eines flüssigen Stromes zugeführt wird.

Im Rahmen einer besonders bevorzugten Ausführungsform führt man das erfindungsgemäße Verfahren in einer Anlage durch, wie sie schematisch in Figur 1 gezeigt ist. Die bevorzugte Ausführungsform zeichnet sich dadurch aus, dass man eine Trennwandkolonne (TK) einsetzt, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist.

Das als Einsatzstoff dienende Neral und Geranial enthaltende Stoffgemisch (a) wird erfindungsgemäß vorzugsweise in den mittleren Bereich des Zulaufteils (2, 4) zugeführt, das Neral in reiner Form mit einem Neral-Gehalt von größer oder gleich 95 Gew. % bezogen auf die Gesamtmenge als Seitenabzug (f) aus dem mittleren Bereich des Entnahmeteils (3, 5) gewonnen und eine oder mehrere Leichtsiederfraktionen aus dem oberen gemeinsamen Kolonnenbereich (1) und eine oder mehrere Hochsiederfraktionen aus dem unteren gemeinsamen Kolonnenbereich (6) abgeführt.

Der Zulaufstrom (a) kann über einen Vorheizer (VH) als flüssiger (b), gasförmiger (c) oder teilweise flüssig und gasförmiger Strom in die Kolonne (TK) eingeleitet werden. Der Kopfstrom der Kolonne wird im Kondensator (K) ganz oder teilweise kondensiert. Bei einer teilweisen Kondensation (Dephlegmator-Betrieb) enthält der Abgasstrom (k) des Kopfkondensators (K) üblicherweise noch merkliche Mengen an kondensierbaren Leichtsiedern, die dann in einem bei niedriger Temperatur betriebenen Nachkondensator niedergeschlagen werden können.

Das im Kondensator (K) niedergeschlagene Kopfprodukt kann in dem Destillatbehälter (DB) gepuffert und über die Rücklaufpumpe (RP) als Kolonnenrücklauf (i) der Kolonne wieder zugeführt werden. Bei Bedarf lässt sich hieraus auch eine Destillatfraktion (j) gewinnen. Bei Intergration des Kondensators in den Kolonnenkopf kann auf den Destillatbehälter (DB) und die Rücklaufpumpe verzichtet (RP) werden.

Der Sumpfstrom wird vorteilhaft über die Umlaufpumpe (UP) dem Sumpfverdampfer (SV) zugeführt, der bevorzugt als Fallfilmverdampfer ausgeführt ist. Diesem Umpumpstrom kann auch der Sumpfaustrag (g) der Kolonne (TK) entnommen werden.

Das Wertprodukt Neral in reiner Form mit einem Neral-Gehalt von größer oder gleich 95 Gew. % bezogen auf die Gesamtmenge wird bevorzugt als flüssiger Seitenabzug, Strom (f), aus dem Entnahmeteil der Trennwandkolonne (TK) abgezogen. Es ist auch möglich, bei Bedarf den Wertprodukt-Strom (f) als gasförmigen Abzug zu entnehmen, dann wird aber üblicherweise ein weiterer Kondensator benötigt.

Der obere gemeinsame Teilbereich (1) der Kolonne weist üblicherweise 5 bis 50%, der Verstärkungsteil (2) des Zulaufteils der Kolonne 5 bis 50%, der Abtriebsteil (4) des Zulaufteils der Kolonne 2 bis 50%, der Abtriebsteil (2) des Entnahmeteils der Kolonne 5 bis 50%, der Verstärkungsteil (5) des Entnahmeteils 2 bis 50%, und der gemeinsame untere Teil (6) der Kolonne 5 bis 50% der Gesamtzahl der theoretischen Trennstufen der Kolonne auf, wobei sich die gewählten Prozentangaben zu 100% addieren.

Bevorzugt weist der obere gemeinsame Teilbereich (1) der Kolonne 10 bis 25%, der Verstärkungsteil (2) des Zulaufteils der Kolonne 15 bis 30%, der Abtriebsteil (4) des Zulaufteils der Kolonne 5 bis 20%, der Abtriebsteil (3) des Entnahmeteils der Kolonne 15 bis 30%, der Verstärkungsteil (5) des Entnahmeteils 5 bis 20%, und der gemeinsame untere Teil (6) der Kolonne 10 bis 25% der Gesamtzahl der theoretischen Trennstufen der Kolonne auf, wobei sich die gewählten Prozentangaben zu 100% addieren.

Die Summe der Zahl der theoretischen Trennstufen der Teilbereiche (2) und (4) im Zulaufteil beträgt bevorzugt 80 bis 110%, besonders bevorzugt 95 bis 105% der Summe der Zahl der Trennstufen der Teilbereiche (3) und (5) im Entnahmeteil.

Vorteilhafterweise sind die Zulaufstelle und die Seitenabzugsstelle hinsichtlich der Lage der theoretischen Trennstufen auf unterschiedlicher Höhe in der Kolonne angeordnet, indem die Zulaufstelle um 1 bis 50, bevorzugt um 30 bis 45 theoretische Trennstufen höher oder niedriger anordnet als die Seitenabzugsstelle.

Es hat sich darüber hinaus als vorteilhaft erwiesen wenn der durch die Trennwand unterteilte Teilbereich der Kolonne bestehend aus den Teilbereichen (2), (3), (4) und (5) oder Teilen davon mit geordneten Packungen oder Füllkörpern (beispielsweise Gewebepackungen wie Montz A3-500, Sulzer BX oder CY) bestückt ist. Weiterhin hat es sich als vorteilhaft erwiesen, wenn die Trennwand in diesen Teilbereichen wärmeisolierend ausgeführt ist.

Der Brüdenstrom am unteren Ende der Trennwand kann durch die Wahl und/oder Dimensionierung der Trenneinbauten und/oder den Einbau druckverlusterzeugender Vorrichtungen, beispielsweise von Blenden, so eingestellt wird, dass das Verhältnis des Brüdenstroms im Zulaufteil zu dem des Entnahmeteils 0,8 bis 1,2, bevorzugt 0,9 bis 1,1 beträgt.

Die aus dem oberen gemeinsamen Teilbereich (1) der Kolonne ablaufende Flüssigkeit wird vorteilhaft in einem in der Kolonne oder außerhalb der Kolonne angeordneten Auffangraum gesammelt und gezielt durch eine Festeinstellung oder Regelung am oberen Ende der Trennwand so aufgeteilt, dass das Verhältnis des Flüssigkeitsstroms zum Zulaufteil zu dem zum Entnahmeteil 0,1 bis 2,0 bei größtenteils flüssigen Zulauf und 1,0 bis 2 bei gasförmigem Zulauf beträgt. Dabei ist der flüssige Zulauf erfindungsgemäß bevorzugt.

Die aus dem oberen gemeinsamen Teilbereich (1) auf den Zulaufteil ablaufende Flüssigkeit kann über eine Pumpe gefördert oder über eine statische Zulaufhöhe von mindestens 1 m mengengeregelt aufgegeben werden, bevorzugt über eine Kaskadenregelung in Verbindung mit der Flüssigkeitsstandregelung des Auffangraums. Die Regelung wird bevorzugt so eingestellt, dass die auf den Zulaufteil aufgegebene Flüssigkeitsmenge nicht unter 30 % des gewünschten Normalwertes sinken kann. Darüber hinaus wird die Aufteilung der aus dem Teilbereich (3) im Entnahmeteil der Kolonne ablaufenden Flüssigkeit auf den Seitenabzug und auf den Teilbereich (5) im Entnahmeteil der Kolonne durch eine Regelung vorteilhaft so eingestellt, dass die auf den Teilbereich (5) aufgegebene Flüssigkeitsmenge nicht unter eine Höhe von 30% des gewünschten Normalwertes sinken kann. Als Normalwert sind dabei vorteilhaft die zwei- bis vierfache Menge, bezogen auf die Zulaufmenge an Geranial/Neral-Gemisch, anzunehmen.

Die Trennwandkolonne weist vorzugsweise am oberen und unteren Ende der Trennwand Probenahmemöglichkeiten auf, aus der Kolonne kontinuierlich oder in zeitlichen Abständen flüssig oder gasförmig Proben entnommen und hinsichtlich ihrer Zusammensetzung, bevorzugt gaschromatographisch, untersucht werden können.

Das Aufteilungsverhältnis der Flüssigkeit am oberen Ende der Trennwand wird vorzugsweise so eingestellt, dass die Konzentration an denjenigen Komponenten der Hochsiederfraktion, für die im Seitenabzug ein bestimmter Grenzwert für die Konzentration erzielt werden soll (speziell Geranial), in der Flüssigkeit am oberen Ende der Trennwand 10 bis 80%, bevorzugt 30 bis 50% des Wertes ausmacht, der im Seitenabzugsprodukt erzielt werden soll. Die Flüssigkeitsaufteilung sollte bevorzugt dahingehend eingestellt werden, dass bei höheren Gehalten an Komponenten der Hochsiederfraktion mehr und bei niedrigeren Gehalten an Komponenten der Hochsiederfraktion weniger Flüssigkeit auf den Zulaufteil geleitet wird.

Die Heizleistung im Verdampfer (SV) stellt man bevorzugt so ein, dass die Konzentration an denjenigen Komponenten der Leichtsiederfraktion, für die im Seitenabzug ein bestimmter Grenzwert für die Konzentration erzielt werden soll (speziell Isocitrale), am unteren Ende der Trennwand 10 bis 80%, bevorzugt 30 bis 50% des Wertes ausmacht, der im Seitenabzugsprodukt erzielt werden soll. Die Heizleistung stellt man vorteilhaft dahingehend ein, dass bei höherem Gehalt an Komponenten der Leichtsiederfraktion die Heizleistung erhöht und bei niedrigerem Gehalt an Komponenten der Leichtsiederfraktion die Heizleistung verringert wird.

Die Destillatentnahme, d.h. die Entnahme der tiefsiedenden Nebenprodukte erfolgt bevorzugt temperaturgeregelt. Als Regeltemperatur verwendet man mit Vorteil eine Messstelle im Teilbereich (1) der Kolonne, die um 3 bis 8, bevorzugt 4 bis 6 theoretische Trennstufen unterhalb des oberen Endes der Kolonne angeordnet ist.

Die Entnahme des Sumpfprodukts erfolgt bevorzugt mengengeregelt, vorzugsweise in Abhängigkeit von der Zulaufmenge.

Die Entnahme des als Seitenprodukt gewonnenen Verfahrensproduktes Neral in reiner Form mit einem Neral-Gehalt von größer oder gleich 95 Gew. % bezogen auf die Gesamtmenge erfolgt bevorzugt standgeregelt wobei als Regelgröße vorzugsweise der Flüssigkeitsstand im Kolonnensumpf verwendet wird.

Der Zulaufstrom (a) wird vorzugsweise teilweise oder vollständig vorverdampft und der Kolonne zweiphasig oder in Form eines gasförmigen und eines flüssigen Stromes zugeführt.

Im Rahmen einer bevorzugten Ausführungsform setzt man eine Trennwandkolonne ein, deren Trennwand nicht in die Kolonne eingeschweißt ist, sondern in Form von lose gesteckten und adäquat abgedichteten Teilsegmenten gestaltet ist.

Die Flüssigkeitsverteilung in den einzelnen Teilbereichen der Kolonne kann bevorzugt gezielt ungleichmäßig eingestellt werden, wobei insbesondere in den Teilbereichen (2) und (5) die Flüssigkeit verstärkt im Wandbereich aufgegeben wird und in den Teilbereichen (3) und (4) die Flüssigkeit reduziert im Wandbereich aufgegeben wird.

Das Verteilungsverhältnis der rücklaufenden Flüssigkeit zwischen Abnahme- und Zulaufseite der Trennwand beträgt vorzugsweise etwa 1 zu 1 bis etwa 3 zu 1 bevorzugt etwa 1 zu 1 bis etwa 1,5 zu 1.

Die Lage der Trennwand in den einzelnen Teilbereichen der Kolonne kann vorteilhaft so angepasst werden, dass die Querschnitte von Zulauf- und Entnahmeteil verschiedene Flächen aufweisen.

Eine besonders bevorzugte Ausführungsform des erfindungsgemäßen Verfahrens zeichnet sich dadurch aus, dass mindestens eine Leichtsiederfraktion als flüssiger oder gasförmiger, bevorzugt als flüssiger Seitenabzug (n) im oberen Abschnitt (1) der Kolonne, bevorzugt 4 bis 10 theoretische Stufen unter dem Kolonnenkopf, gewonnen wird (siehe Figur 2). In diesem Fall ist es zweckmäßig, den obere Kolonnenabschnitt (1) in zwei Abschnitte ((1a) und (1 b)) aufzuteilen. Zwischen diesen Abschnitten kann durch einen geeigneten Sammler die aus dem Abschnitt (1a) ablaufende Flüssigkeit aufgefangen und wieder auf den unterhalb liegenden Abschnitt (1 b) verteilt werden (siehe Figur 2). Aus dem Sammler kann eine Leichtsieder- arme und Neral-arme Fraktion abgezogen werden, die vor allem isomere Citrale enthält.

Diese durch den zusätzliche Seitenabzug (n) zugängliche iso-Citral-reiche Nebenproduktfraktion kann in geeigneter Weise weiterverwertet werden, beispielsweise kann sie einer durchgreifenden Hydrierung oder einer partiellen Hydrierung zum Tetrahydrogeraniol unterworfen werden, wodurch zu entsorgende Abfall- bzw. Nebenprodukte vermieden werden können.

Eine erfindungsgemäß besonders bevorzugte Ausführungsform betrifft daher ein kontinuierliches Verfahren zur Herstellung von Neral der Formel (I) in reiner Form mit einem Neral-Gehalt von größer oder gleich 95 Gew. % bezogen auf die Gesamtmenge durch destillative Abtrennung von Neral aus Stoffgemischen enthaltend Neral und Geranial der Formel (II)
- wobei man die destillative Abtrennung in einer Trennwandkolonne (TK), die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist, mit 80 bis 200 theoretischen Trennstufen und mehrerer, bevorzugt 2 bis 4, besonders bevorzugt 2 oder 3 Seitenabzugsstellen bei einem absoluten Betriebsdruck von 5 bis 200 mbar durchführt und
- wobei man Neral in reiner Form mit einem Neral-Gehalt von größer oder gleich 95 Gew. % bezogen auf die Gesamtmenge_als Seitenabzug (f) aus dem mittleren Bereich des Entnahmeteils (3, 5) gewinnt und
- wobei man eine Leichtsiederfraktion (n) als flüssigen oder gasförmigen, bevorzugt als flüssigen Seitenabzug aus dem oberen gemeinsamen Kolonnenbereich (1) gewinnt.

Eine weitere bevorzugte Ausführungsform des erfindungsgemäßen Verfahrens zeichnet sich dadurch aus, dass mindestens eine Hochsiederfraktion als gasförmiger Seitenabzug (o) im unteren gemeinsamen Teilbereich der Kolonne (6), bevorzugt 1 bis 5 theoretische Stufen über dem Kolonnensumpf, gewonnen wird (siehe Figur 2). Dadurch kann ein besonders hochsiederarmes Geranial-reiches Produkt erhalten werden. In diesem Fall kann es zweckmäßig sein, den unteren Kolonnenabschnitt (6) in zwei Abschnitte (6a und 6b) aufzuteilen. Zwischen diesen Abschnitten kann durch einen geeigneten Sammler die aus dem Abschnitt (6a) ablaufende Flüssigkeit aufgefangen und wieder auf den unterhalb liegenden Abschnitt (6b) verteilt (siehe Figur 2) sowie der Gasstrom für den Seitenabzug entnommen werden.

Als Sumpfverdampfer (SV) für die Trennwandkolonne kann vorteilhaft ein Dünnschichtapparat, beispielsweise ein Fallfilmverdampfer, verwendet werden.

Der Kopfkondensator (K) kann beispielsweise als Plattenapparat ausgeführt und in den Kolonnenmantel integriert sein.

Das erfindungsgemäß zugängliche Neral in reiner Form mit einem Neral-Gehalt von größer oder gleich 95 Gew. % bezogen auf die Gesamtmenge wird über den Seitenabzug, bzw. im Fall, dass weitere Seitenabzüge vorgesehen sind, über den mittleren Seitenabzug (f) kontinuierlich gewonnen und weist im Rahmen einer bevorzugten Ausführungsform einen Neral-Gehalt von über 98 Gew.-%, bevorzugt von 98,5 bis 99,5 Gew.-%, einen Geranial-Gehalt von weniger als 0,3 Gew.-% und einen Gehalt an sonstigen Isomeren (Citral-Isomere der Formeln (III), (IV) und (V)) von weniger als 1 Gew.-% (jeweils bezogen auf die Gesamtmenge des erhaltenen Gemischs), gegebenenfalls neben geringen Mengen weiterer Verunreinigungen, auf.

Wird ein wie vorstehend beschriebener oberer Seitenabzug (n) vorgesehen, kann dort ein Nebenproduktgemische erhalten werden, das üblicherweise einen Neral-Gehalt von weniger als 80 Gew.-%, einen Geranial-Gehalt von weniger als 0,1 Gew.-% und einen Gehalt an sonstigen Isomeren, insbesondere der Citral-Isomere der Formeln (III), (IV) und (V) von über 20 Gew.-%, bevorzugt von über 30 Gew.-% aufweist. Daneben kann in einem gewünschtenfalls vorgesehenen unteren Seitenabzug (o), genauso wie in der Sumpffraktion (g) ein Produktgemisch mit einem Neral-Gehalt von weniger als 20 Gew.-% und einem Geranial-Gehalt von mehr als 70 Gew.-% erhalten werden. Die Kopffraktion (j) weist üblicherweise einen Neral-Gehalt von weniger 30 Gew.-% auf. Die davon abgetrennte Leichtsiederfraktion (k) weist üblicherweise einen Neral-Gehalt von weniger als 5 Gew.-% neben iso-Citralen als Hauptkomponenten auf.

Die vorliegende Erfindung betrifft auch die Verwendung einer wie vorstehend beschriebenen Trennwandkolonne oder einer Zusammenschaltung von zwei Destillationskolonnen in Form einer thermischen Kopplung, bevorzugt einer Trennwandkolonne mit 80 bis 200 theoretischen Trennstufen und einer oder mehrerer Seitenabzugsstellen zur kontinuierlichen Herstellung von Neral der Formel (I) in reiner Form mit einem Neral-Gehalt von größer oder gleich 95 Gew. % bezogen auf die Gesamtmenge durch destillative Abtrennung von Neral aus Stoffgemischen enthaltend Neral und Geranial der Formel (II) bzw. die Verwendung derselben zur Isolierung von Neral. Darüber hinaus betrifft die vorliegende Erfindung auch eine wie vorstehend beschriebene Trennwandkolonne oder eine Zusammenschaltung von zwei Destillationskolonnen in Form einer thermischen Kopplung, bevorzugt eine Trennwandkolonne mit 80 bis 200 theoretischen Trennstufen und einer oder mehrerer Seitenabzugsstellen, die sich zur kontinuierlichen Herstellung von Neral der Formel (I) in reiner Form mit einem Neral-Gehalt von größer oder gleich 95 Gew. % bezogen auf die Gesamtmenge durch destillative Abtrennung von Neral aus Stoffgemischen enthaltend Neral und Geranial der Formel (II) eignet.

Die folgenden Beispiele dienen der Erläuterung der Erfindung, ohne sie in irgendeiner Weise zu beschränken:

### Beispiel 1:

Die für die folgenden Beispiele verwendete Trennwandkolonne wurde aus fünf je 1,2 m langen Glasschüssen mit 64 mm Innendurchmesser aufgebaut. In die drei mittleren Schüsse wurde eine Trennwand aus Metallblech gesteckt. Unterhalb und oberhalb des Trennwandbereiches wurden Laborpackungen (Sulzer CY) und im Trennwandbereich Metall-Geweberinge aus Edelstahl mit 5 mm Durchmesser eingebaut. Bei Trennleistungsmessungen, die mit dem Xylol-Isomerengemischen bei einem Kopfdruck von 60 mbar durchgeführt wurden, konnte eine Gesamttrennleistung von 100 theoretischen Böden über die gesamte Kolonne und etwa 55 theoretischen Böden im Trennwandbereich gemessen werden. Die Gesamtzahl der vorhandenen theoretischen Böden betrug also in etwa 155. Die Kolonne wurde mit einem ölbeheizten Dünnschichtverdampfer (0,1 m²) und einem mit Kühlwasser gekühlten Kondensator ausgerüstet.

Temperaturen an verschiedenen Höhen in der Kolonne sowie der Kopfdruck und der Druckverlust über die Kolonne wurden mittels eines Messwerterfassungssystem gemessen. Die Kolonne verfügte über Durchflussmessungen in den Zu- und Abläufen sowie eine Mengenmessung des Rücklaufs, die als Regelgröße für die Vorlauftemperatur des Ölthermostaten diente. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.

Der Kolonne wurde in einer Höhe von 136 cm zum Zulaufteil der Trennwand 461 g/h eines auf 110°C vorgewärmten flüssigen Gemisches aus 48,7 GC-Flächen % Neral, 47,8 GC-Flächen % Geranial und 1,4 GC-Flächen % andere Citral-Isomere zugeführt. Die Kolonne wurde bei 10 mbar Kopfdruck und einem Rücklauf von 2,5 kg/h betrieben. Dabei stellte sich ein Druckverlust von etwa 34 mbar (± 1 mbar) ein. Am Kopf der Kolonne wurde eine Temperatur von 82,3°C und im Sumpf eine Temperatur von 128,4 °C (± 0,5 K) gemessen. Der Sumpfabzug wurde über eine Waagensteuerung fest auf 240 g/h und die Destillatabnahme auf 20 g/h (± 1 g/h) eingestellt. Das Rücklaufverhältnis betrug somit etwa 125 : 1. Die Flüssigkeit wurde oberhalb der Trennwand in einem Verhältnis von 1 : 1,1 (Zulauf- : Entnahmeteil) aufgeteilt. In einer Höhe von 490 cm im Abnahmeteil der Trennwand wurde ein gasförmiger Seitenabzug (f) entnommen und in einem Glaskühler kondensiert, aus dem in Abhängigkeit vom Sumpffüllstand etwa 200 g/h Reinprodukt über eine Pumpe abgezogen wurde.

Die gewonnenen Fraktionen wurden gaschromatographisch mit Hilfe eines Standard-GC analysiert. Gaschromatographische Analysen wurden nach der folgenden Methode durchgeführt:
25m OV-1, ID.: 0,32 mm, FD.: 0,31 µm; 50 °C / 2 min - 10 °C/min auf 150 °C, 5 min - 20 °C/min auf 280 °C /15 min; t_{R} (Citral-Isomer III): 10,4 min; t_{R} (Citral-Isomer IV): 10,7 min; t_{R} (Citral-Isomer V): 11,0 min; t_{R} (Neral I): 12,3 min; t_{R} (Geranial II): 12,6 min

Das am Seitenabzug gewonnene Reinprodukte enthielt neben 98,5 GC-Flächen % Neral auch 0,3 GC-Flächen % Geranial und 0,65 GC-Flächen % andere Citralisomere. Im Sumpfabzug wurden durch GC-Analyse 92,5 GC-Flächen % Geranial und 6,8 GC-Flächen % Neral bestimmt, das Destillat enthielt 32,1 GC-Flächen % Neral und 39,6 GC-Flächen % andere Citral-Isomere.

### Beispiel 2:

Die im Beispiel 1 beschriebene Kolonne wurde durch einen weiteren, gasförmigen Seitenabzug (n) im oberen gemeinsamen Kolonnenteil (1), bei etwa 590 cm Höhe, ergänzt, der wiederum mit einem Seitenkondensator versehen wurde. Über eine Waagensteuerung wurde dort eine Abnahmemenge von 15 g/h (± 1 g/h) eingestellt.

Der Kolonne wurde in einer Höhe von 136 cm zum Zulaufteil der Trennwand 460 g/h eines auf 110°C vorgewärmten flüssigen Gemisches aus 50,2 GC-Flächen % Neral, 47,2 GC-Flächen % Geranial und 0,9 GC-Flächen % anderer Citral-Isomere zugeführt. Die Kolonne wurde bei 10 mbar Kopfdruck und einem Rücklauf von 2,5 kg/h betrieben. Dabei stellte sich ein Druckverlust von etwa 37 mbar (± 1 mbar) ein. Am Kopf der Kolonne wurde eine Temperatur von 68,8°C und im Sumpf eine Temperatur von 130,1 °C (± 0,5 K) gemessen. Der Sumpfabzug wurde über eine Waagensteuerung fest auf 240 g/h und die Destillatabnahme auf etwa 3 g/h (± 1 g/h) eingestellt. Das Rücklaufverhältnis betrug somit etwa 600 bis 1200 : 1. Die Flüssigkeit wurde oberhalb der Trennwand in einem Verhältnis von 1:1,1 (Zulauf- : Entnahmeteil) aufgeteilt. In einer Höhe von 490 cm im Abnahmeteil der Trennwand wurde wiederum ein gasförmiger Seitenabzug (f) entnommen und in einem Glaskühler kondensiert, aus dem in Abhängigkeit vom Sumpffüllstand etwa 200 g/h Reinprodukt über eine Pumpe abgezogen wurde.

Das am Seitenabzug (f) gewonnene Reinprodukt enthielt neben 98,5 GC.-Flächen % Neral auch 0,3 GC.-Flächen % Geranial und 0,5 GC.-Flächen % andere Citralisomere. Der obere Seitenabzug enthielt neben 55,5 GC.-Flächen % Neral 29,5 GC.-Flächen % andere Citral-Isomere. Im Sumpfabzug wurden durch GC-Analyse 90,3 GC.-Flächen % Geranial und 8,9 GC.-Flächen % Neral bestimmt, das Destillat enthielt nur Spuren von Neral und 48,5 GC.-Flächen % andere Citral-Isomere.

### Vergleichsbeispiel:

In einer mit 6 m Sulzer CY-Packung ausgerüsteten einfachen Glas-Laborkolonne ohne Trennwand und ohne Seitenabzug (theoretische Stufenzahl von etwa 90) mit einem Innendurchmesser von 50 mm wurde ein Gemisch aus 50,2 GC.-Flächen % Neral, 47 GC.-Flächen % Geranial und 1,3 GC.-Flächen % andere Citral-Isomere bei 5 mbar Kopfdruck kontinuierlich destilliert. Die Zulaufmenge betrug 500 g/h, am Sumpf wurden 250 g/h ausgetragen. Der Druckverlust über die Kolonne betrug bei einem Rücklaufverhältnis von 11 : 1 etwa 28 mbar, die Sumpftemperatur 121 °C und die Kopftemperatur 81 °C.

Am Kopfkondensator wurden bei etwa 20°C ca. 250 g/h eines flüssigen Destillats mit einem Neral-Gehalt von 88,1 GC.-Flächen % und einem Geranial-Gehalt von 2,7 GC.-Flächen % gewonnen, der Gehalt an anderen Citral-Isomeren im Destillat betrug in Summe 7,0 GC.-Flächen %, was auf eine merkliche Bildung dieser Isomere unter Destillationsbedingungen schließen lässt.

## Patentansprüche

1. Kontinuierliches Verfahren zur Herstellung von Neral der Formel (I) 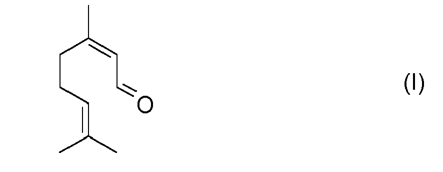 in reiner Form mit einem Neral-Gehalt von größer oder gleich 95 Gew. % bezogen auf die Gesamtmenge durch destillative Abtrennung von Neral aus Stoffgemischen enthaltend Neral und Geranial der Formel (II) 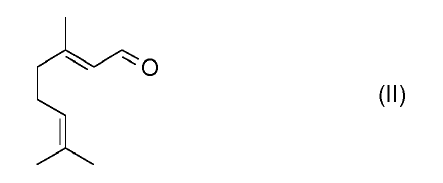 wobei man die destillative Abtrennung in einer Trennwandkolonne oder in einer Zusammenschaltung von zwei Destillationskolonnen in Form einer thermischen Kopplung mit 80 bis 200 theoretischen Trennstufen und einer oder mehrerer Seitenabzugsstellen bei einem absoluten Betriebsdruck von 5 bis 200 mbar durchführt, **dadurch gekennzeichnet, dass** man ein Stoffgemisch einsetzt, dass zu 30 bis 70 Gew. % aus Neral, zu 70 bis 30 Gew.-% aus Geranial und zu 0 bis 5 Gew.-% aus weiteren Komponenten besteht, wobei sich die Prozentangaben zu 100 Gew.-% addieren.

2. Verfahren nach Anspruch 1, **dadurch gekennzeichnet, dass** man die destillative Abtrennung so durchführt, dass man das eingesetzte Neral und Geranial enthaltende Stoffgemisch in jeweils eine oder mehrere Leichtsieder-, Mittelsieder- und Schwersiederfraktion bzw. -fraktionen auftrennt und Neral in reiner Form mit einem Neral-Gehalt von größer oder gleich 95 Gew. % bezogen auf die Gesamtmenge als Mittelsiederfraktion an der Seitenabzugsstelle in flüssiger oder gasförmiger Form entnommen wird.

3. Verfahren nach Anspruch 1 oder 2, **dadurch gekennzeichnet, dass** man die Trennwandkolonne oder die Zusammenschaltung von zwei Destillationskolonnen in Form einer thermischen Kopplung mit einem absoluten Kopfdruck von 10 bis 40 mbar und mit einem absoluten Sumpfdruck von 20 bis 50 mbar betreibt.

4. Verfahren nach einem der Ansprüche 1 bis 3, **dadurch gekennzeichnet, dass** man eine Trennwandkolonne (TK) einsetzt, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist und dass der durch die Trennwand (T) unterteilte Teilbereich der Kolonne bestehend aus den Teilbereichen (2), (3), (4) und (5) oder Teilen davon mit geordneten Packungen oder Füllkörpern bestückt ist.

5. Verfahren nach Anspruch 4, **dadurch gekennzeichnet, dass** man das Neral und Geranial enthaltende Stoffgemisch (a) in den mittleren Bereich des Zulaufteils (2, 4) zuführt, das Neral in reiner Form mit einem Neral-Gehalt von größer oder gleich 95 Gew. % bezogen auf die Gesamtmenge als Seitenabzug aus dem mittleren Bereich des Entnahmeteils (3, 5) gewinnt und eine oder mehrere Leichtsiederfraktionen aus dem oberen gemeinsamen Kolonnenbereich (1) und eine oder mehrere Hochsiederfraktionen aus dem unteren gemeinsamen Kolonnenbereich (6) abführt.

6. Verfahren nach einem der Ansprüche 1 bis 5, **dadurch gekennzeichnet, dass** mindestens eine Hochsiederfraktion als gasförmiger oder flüssiger Seitenabzug im unteren gemeinsamen Kolonnenbereich (6) gewonnen wird.

7. Verfahren nach einem der Ansprüche 1 bis 6, **dadurch gekennzeichnet, dass** mindestens eine Leichtsiederfraktion als flüssiger Seitenabzug im oberen gemeinsamen Kolonnenbereich (1) gewonnen wird.

8. Verfahren nach einem der Ansprüche 1 bis 7 zur Herstellung von Neral der Formel (I) in reiner Form durch destillative Abtrennung von Neral aus Stoffgemischen enthaltend Neral und Geranial der Formel (II)
- wobei man die destillative Abtrennung in einer Trennwandkolonne (TK), die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist, mit 80 bis 200 theoretischen Trennstufen und mehrerer, bevorzugt 2 bis 4, besonders bevorzugt 2 oder 3 Seitenabzugsstellen bei einem absoluten Betriebsdruck von 5 bis 200 mbar durchführt und
- wobei man Neral in reiner Form als Seitenabzug (f) aus dem mittleren Bereich des Entnahmeteils (3, 5) gewinnt und
- wobei man eine Leichtsiederfraktion (n) als flüssigen oder gasförmigen, bevorzugt als flüssigen Seitenabzug aus dem oberen gemeinsamen Teilbereich (1) der Trennwandkolonne gewinnt.

9. Verfahren nach einem der Ansprüche 1 bis 8, **dadurch gekennzeichnet, dass** die Zulaufstelle und die Seitenabzugsstelle hinsichtlich der Lage der theoretischen Trennstufen auf unterschiedlicher Höhe in der Kolonne angeordnet sind, indem die Zulaufstelle um 1 bis 50 theoretische Trennstufen höher oder niedriger angeordnet ist als die Seitenabzugsstelle.

10. Verfahren nach einem der Ansprüche 1 bis 9, **dadurch gekennzeichnet, dass** der durch die Trennwand unterteilte Teilbereich der Kolonne bestehend aus den Teilbereichen (2), (3), (4) und (5) oder Teilen davon mit geordneten Packungen oder Füllkörpern bestückt ist und/oder die Trennwand in diesen Teilbereichen wärmeisolierend ausgeführt ist.

11. Verfahren nach einem der Ansprüche 1 bis 10, **dadurch gekennzeichnet, dass** der Brüdenstrom am unteren Ende der Trennwand durch die Wahl und/oder Dimensionierung der Trenneinbauten und/oder den Einbau druckverlusterzeugender Vorrichtungen so eingestellt wird, dass das Verhältnis des Brüdenstroms im Zulaufteil zu dem des Entnahmeteils 0,8 bis 1,2 beträgt.

12. Verfahren nach einem der Ansprüche 1 bis 11, **dadurch gekennzeichnet, dass** die aus dem oberen gemeinsamen Teil (1) der Kolonne ablaufende Flüssigkeit in einem in der Kolonne oder außerhalb der Kolonne angeordneten Auffangraum gesammelt und gezielt durch eine Festeinstellung oder Regelung am oberen Ende der Trennwand so aufgeteilt wird, dass das Verhältnis des Flüssigkeitsstroms zum Zulaufteil zu dem zum Entnahmeteil 0,1 bis 2,0 bei größtenteils flüssigen Zulauf und 1,0 bis 2 bei gasförmigem Zulauf beträgt.

13. Verfahren nach einem der Ansprüche 1 bis 12, **dadurch gekennzeichnet, dass** die Destillatentnahme temperaturgeregelt erfolgt und als Regeltemperatur eine Messstelle im Teilbereich (1) der Kolonne verwendet wird, die um 3 bis 8 theoretische Trennstufen unterhalb des oberen Endes der Kolonne angeordnet ist.

14. Verfahren nach einem der Ansprüche 1 bis 13, **dadurch gekennzeichnet, dass** die Entnahme des Sumpfprodukts mengengeregelt in Abhängigkeit von der Zulaufmenge und dass die Entnahme des Seitenprodukts standgeregelt erfolgt, wobei als Regelgröße der Flüssigkeitsstand im Kolonnensumpf verwendet wird.

15. Verfahren nach einem der Ansprüche 1 bis 14, **dadurch gekennzeichnet, dass** als Sumpfverdampfer (SV) ein Dünnschichtapparat verwendet wird und / oder der Kopfkondensator (K) als Plattenapparat ausgeführt ist und in den Kolonnenmantel integriert wird.

## Claims

1. A continuous process for preparing neral of the formula (I) 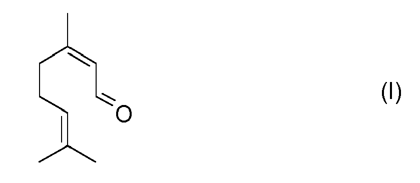 in pure form having a content of greater than or equal to 95 % by weight based on the total amount by distillative separation of neral from substance mixtures comprising neral and geranial of the formula (II) 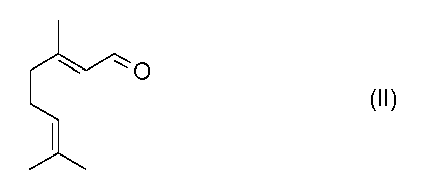 wherein the distillative separation is performed in a dividing wall column or in a connection of two distillation columns in the form of a thermal coupling with from 80 to 200 theoretical plates and one or more side draws at an absolute operating pressure of from 5 to 200 mbar, **characterized in that** a substance mixture is used which consists to an extent of from 30 to 70% by weight of neral, to an extent of from 70 to 30% by weight of geranial and to an extent of from 0 to 5% by weight of further components, where the percentages add up to 100% by weight.

2. The process according to claim 1, wherein the distillative separation is performed in such a way that the substance mixture comprising neral and geranial used is separated into in each case one or more low boiler, medium boiler and high boiler fraction(s) and neral is withdrawn in pure form having a content of greater than or equal to 95 % by weight based on the total amount as a medium boiler fraction at the side draw in liquid or gaseous form.

3. The process according to claim 1 or 2, wherein the dividing wall column or the connection of two distillation columns in the form of a thermal coupling is operated at an absolute top pressure of from 10 to 40 mbar and at an absolute bottom pressure of from 20 to 50 mbar.

4. The process according to any one of claims 1 to 3, wherein a dividing wall column (TK) is used, which has a dividing wall (T) in the longitudinal direction of the column to form an upper combined column region (1), a lower combined column region (6), a feed section (2, 4) with rectifying section (2) and stripping section (4) and a withdrawal section (3, 5) with stripping section (3) and rectifying section (5), and wherein the subregion of the column which is divided by the dividing wall (T) and consists of the subregions (2), (3), (4) and (5) or parts thereof is equipped with structured packings or random packings.

5. The process according to claim 4, wherein the substance mixture (a) which comprises neral and geranial is fed into the middle region of the feed section (2, 4), the neral is obtained in pure form having a content of greater than or equal to 95 % by weight based on the total amount as a side draw from the middle region of the withdrawal section (3, 5) and one or more low boiler fractions are removed from the upper combined column region (1) and one or more high boiler fractions from the lower combined column region (6).

6. The process according to any one of claims 1 to 5, wherein at least one high boiler fraction is obtained as a gaseous or liquid side draw in the lower combined column region (6).

7. The process according to any one of claims 1 to 6, wherein at least one low boiler fraction is obtained as a liquid side draw in the upper combined column region (1).

8. The process according to any one of claims 1 to 7 for preparing neral of the formula (I) in pure or enriched form by distillative separation of neral from substance mixtures comprising neral and geranial of the formula (II),
- the distillative removal being performed in a dividing wall column (TK) which has a dividing wall (T) in the longitudinal direction of the column to form an upper combined column region (1), a lower combined column region (6), a feed section (2, 4) with rectifying section (2) and stripping section (4), and a withdrawal section (3, 5) with stripping section (3) and rectifying section (5), having from 80 to 200 theoretical plates and a plurality of, preferably from 2 to 4, more preferably 2 or 3, side draw points at an absolute operating pressure of from 5 to 200 mbar, and
- neral being obtained in pure or enriched form as a side draw (f) from the middle region of the withdrawal section (3, 5) and
- a low boiler fraction (n) being obtained as a liquid or gaseous side draw, preferably as a liquid side draw, from the upper combined subregion (1) of the dividing wall column.

9. The process according to any one of claims 1 to 8, wherein the feed point and the side draw point, with regard to the position of the theoretical plates, are arranged at different heights in the column, by virtue of the feed point being arranged from 1 to 50 theoretical plates higher or lower than the side draw point.

10. The process according to any one of claims 1 to 9, wherein the subregion of the column divided by the dividing wall, consisting of subregions (2), (3), (4) and (5) or parts thereof, is equipped with structured packings or random packings and/or the dividing wall is configured with thermal insulation in these subregions.

11. The process according to any one of claims 1 to 10, wherein the vapor stream at the lower end of the dividing wall is adjusted through the selection and/or dimensions of the separating internals and/or the incorporation of devices which generate a pressure drop such that the ratio of the vapor stream in the feed section to that of the withdrawal section is from 0.8 to 1.2.

12. The process according to any one of claims 1 to 11, wherein the liquid effluxing from the upper combined section (1) of the column is collected in a collecting space arranged within the column or outside the column and divided in a controlled manner by a fixed or regulable setting at the upper end of the dividing wall such that the ratio of the liquid stream to the feed section relative to that to the withdrawal section is from 0.1 to 2.0 in the case of a principally liquid feed and from 1.0 to 2 in the case of a gaseous feed.

13. The process according to anyone of claims 1 to 12, wherein the distillate is withdrawn under temperature control and the control temperature used is a measurement point in the subregion (1) of the column, which is arranged from 3 to 8 theoretical plates below the upper end of the column.

14. The process according to any one of claims 1 to 13, wherein the bottom product is withdrawn under quantitative control depending on the feed rate and wherein the side product is withdrawn under level control, the control parameter used being the liquid level in the column bottom.

15. The process according to any one of claims 1 to 14, wherein the bottom evaporator (SV) used is a thin-film apparatus and/or the top condenser (K) is configured as a plate apparatus and integrated into the column jacket.

## Revendications

1. Procédé continu pour la préparation de néral de formule (I) 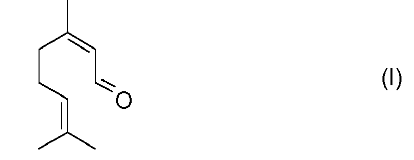 sous forme pure, présentant une teneur en néral supérieure ou égale à 95% en poids, par rapport à la quantité totale, par séparation par distillation de néral de mélanges de substances contenant du néral et du géranial de formule (II) 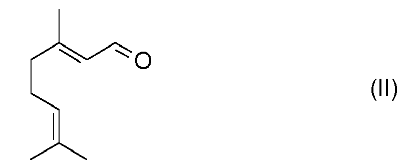 la séparation par distillation étant réalisée dans une colonne à paroi de séparation ou dans une commutation d'au moins deux colonnes de distillation sous forme d'un couplage thermique, présentant 80 à 200 étages théoriques de séparation et présentant un ou plusieurs sites de soutirage latéral, à une pression absolue d'exploitation de 5 à 200 mbars, **caractérisé en ce qu'**on utilise un mélange de substances qui est constitué à raison de 30 à 70% en poids par du néral, à raison de 70 à 30% en poids par du géranial et à raison de 0 à 5% en poids d'autres composants, la somme des indications en pour cent valant 100% en poids.

2. Procédé selon la revendication 1, **caractérisé en ce qu'**on réalise la séparation par distillation de manière telle qu'on sépare le mélange de substances utilisé, contenant du néral et du géranial, à chaque fois en une ou plusieurs fraction(s) de bas point d'ébullition, de point d'ébullition moyen et de point d'ébullition élevé et on prélève le néral sous forme pure, à une teneur en néral supérieure ou égale à 95% en poids, par rapport à la quantité totale, comme fraction à point d'ébullition moyen au niveau du site de soutirage latéral, sous forme liquide ou gazeuse.

3. Procédé selon la revendication 1 ou 2, **caractérisé en ce qu'**on exploite la colonne à paroi de séparation ou la commutation d'au moins deux colonnes de distillation sous forme d'un couplage thermique à une pression absolue de tête de 10 à 40 mbars et à une pression absolue du fond de 20 à 50 mbars.

4. Procédé selon l'une quelconque des revendications 1 à 3, **caractérisé en ce qu'**on utilise une colonne à paroi de séparation (TK), qui présente une paroi de séparation (T) dans la direction longitudinale de la colonne avec formation d'une zone de colonne (1) commune supérieure, d'une zone de colonne (6) commune inférieure, d'une partie d'alimentation (2, 4), présentant une partie d'enrichissement (2) et une partie d'épuisement (4), ainsi que d'une partie de prélèvement (3, 5), présentant une partie d'épuisement (3) et une partie d'enrichissement (5), et **en ce que** la zone partielle de la colonne, sous-divisée par la paroi de séparation (T), constituée par les zones partielles (2), (3), (4) et (5), ou des parties de celles-ci, est équipée de garnissages structurés ou de corps de remplissage structurés.

5. Procédé selon la revendication 4, **caractérisé en ce qu'**on alimente le mélange de substances (a) contenant le néral et le géranial dans la zone centrale de la partie d'alimentation (2, 4), on obtient le néral sous forme pure, à une teneur en néral supérieure ou égale à 95% en poids, par rapport à la quantité totale, comme soutirage latéral de la zone centrale de la partie de prélèvement (3, 5) et on évacue une ou plusieurs fractions à bas point d'ébullition de la zone de colonne (1) commune supérieure et une ou plusieurs fractions à point d'ébullition élevé de la zone de colonne (6) commune inférieure.

6. Procédé selon l'une quelconque des revendications 1 à 5, **caractérisé en ce qu'**on obtient au moins une fraction à point d'ébullition élevé sous forme de soutirage latéral gazeux ou liquide dans la zone de colonne (6) commune inférieure.

7. Procédé selon l'une quelconque des revendications 1 à 6, **caractérisé en ce qu'**on obtient au moins une fraction à bas point d'ébullition sous forme de soutirage latéral liquide dans la zone de colonne (1) commune supérieure.

8. Procédé selon l'une quelconque des revendications 1 à 7 pour la préparation de néral de formule (I) sous forme pure par séparation par distillation de néral de mélanges de substances contenant du néral et du géranial de formule (II)
- la séparation par distillation étant réalisée dans une colonne à paroi de séparation (TK), qui présente une paroi de séparation (T) dans la direction longitudinale de la colonne avec formation d'une zone de colonne (1) commune supérieure, d'une zone de colonne (6) commune inférieure, d'une partie d'alimentation (2, 4), présentant une partie d'enrichissement (2) et une partie d'épuisement (4), ainsi que d'une partie de prélèvement (3, 5), présentant une partie d'épuisement (3) et une partie d'enrichissement (5), présentant 80 à 200 étages théoriques de séparation et plusieurs, de préférence 2 à 4, de manière particulièrement préférée 2 ou 3, sites de soutirage latéral à une pression absolue d'exploitation de 5 à 200 mbars et
- le néral étant obtenu sous forme pure comme soutirage latéral (f) à partir de la zone centrale de la partie de prélèvement (3, 5) et
- une fraction à bas point d'ébullition (n) étant obtenue en tant que soutirage latéral liquide ou gazeux, de préférence liquide, de la zone partielle (1) commune supérieure de la colonne à paroi de séparation.

9. Procédé selon l'une quelconque des revendications 1 à 8, **caractérisé en ce que** le site d'alimentation et le site de soutirage latéral, par rapport à la position des étages théoriques de séparation, sont disposés à une hauteur différente dans la colonne, **en ce que** le site d'alimentation est disposé 1 à 50 étages théoriques de séparation plus haut ou plus bas que le site de soutirage latéral.

10. Procédé selon l'une quelconque des revendications 1 à 9, **caractérisé en ce que** la zone partielle, sous-divisée par la paroi de séparation de la colonne, constituée par les zones partielles (2), (3), (4) et (5), ou des parties de celles-ci, est équipée de garnissages structurés ou de corps de remplissage structurés et/ou la paroi de séparation est réalisée, dans ces zones partielles, de manière thermiquement isolante.

11. Procédé selon l'une quelconque des revendications 1 à 10, **caractérisé en ce que** le flux de vapeur est réglé, au niveau de l'extrémité inférieure de la paroi de séparation, par le choix et/ou par le dimensionnement des inserts de séparation et/ou par le montage de dispositifs générant des pertes de charges, de manière telle que le rapport du flux de vapeur dans la partie d'alimentation à celui de la partie de prélèvement vaut 0,8 à 1,2.

12. Procédé selon l'une quelconque des revendications 1 à 11, **caractérisé en ce que** le liquide qui s'écoule de la partie (1) commune supérieure de la colonne est rassemblé dans un espace de collecte disposé dans la colonne ou en dehors de la colonne et est réparti de manière ciblée, par un réglage fixe ou une régulation au niveau de l'extrémité supérieure de la paroi de séparation, de manière telle que le rapport du flux de liquide vers la partie d'alimentation à celui vers la partie de prélèvement vaut 0,1 à 2,0 dans le cas d'une alimentation en grande partie liquide et 1,0 à 2 dans le cas d'une alimentation gazeuse.

13. Procédé selon l'une quelconque des revendications 1 à 12, **caractérisé en ce que** le prélèvement du distillat a lieu de manière régulée en température et on utilise, comme température de régulation, un site de mesure dans la zone partielle (1) de la colonne qui est disposé 3 à 8 étages théoriques de séparation au-dessous de l'extrémité supérieure de la colonne.

14. Procédé selon l'une quelconque des revendications 1 à 13, **caractérisé en ce que** le prélèvement du produit de fond a lieu de manière régulée en quantité en fonction de la quantité d'alimentation et **en ce que** le prélèvement du produit latéral a lieu de manière régulée en niveau, en utilisant comme grandeur de régulation le niveau de liquide dans le fond de la colonne.

15. Procédé selon l'une quelconque des revendications 1 à 14, **caractérisé en ce qu'**on utilise, comme évaporateur du fond (SV), un appareil à couche mince et/ou le condenseur de tête (K) est conçu sous forme d'appareil à plaques et est intégré dans l'enveloppe de la colonne.
